# DEMANDE DE BREVET EUROPEEN

(11) **EP 1 338 257 A1**
(43) Date de publication de la demande: **27.08.2003**
(21) Numéro de dépôt: 03290109.2
(22) Date de dépôt: 16.01.2003
(51) Int. Cl.: A61F 2/44

(54) **Cage intervertébrale**

(30) Priorité: 28.01.2002 FR 0200956
(71) Demandeur: Razian, Hassan, 94240 l'Hay les Roses (FR)
(72) Inventeur: Razian, Hassan, 94240 l'Hay les Roses (FR)
(74) Mandataire: Flavenot, Bernard

(57) **Abrégé**

La présente invention concerne les cages intervertébrales, notamment pour le traitement du rachis dégénératif.

La cage intervertébrale selon l'invention se caractérise essentiellement par le fait qu'elle comporte une entretoise 1 comportant deux faces de base opposées 3, 4 et une paroi latérale 5 reliant les deux faces de base, l'entretoise étant apte à être disposée entre les faces en regard 8, 9 des deux corps vertébraux 10, 11, une lame 20, et des moyens 21 pour déplacer la lame par rapport à l'entretoise de façon que la lame soit apte à prendre une première position dans laquelle elle est entièrement située dans l'espace 22 compris entre les deux premier et second plans 23, 24 et une seconde position dans laquelle les deux extrémités opposées 25, 26 de cette lame 20 émergent de part et d'autre de l'espace 22, la lame comportant une surface d'épaulement 27, 28 bordant au moins partiellement chacune de ses deux extrémités 25, 26.

## Description

La présente invention concerne les cages intervertébrales trouvant une application particulièrement avantageuse, mais non exclusivement, pour le traitement du rachis dégénératif.

Une cage intervertébrale, notamment pour le traitement du rachis dégénératif, est déjà connue. Elle comporte essentiellement une entretoise en forme de disque comprenant deux faces de base opposées sensiblement planes et parallèles et une paroi latérale reliant les deux faces de base. Cette entretoise est apte à être disposée entre les faces en regard des deux corps vertébraux respectivement de deux vertèbres consécutives, en remplacement du disque endommagé situé entre ces deux vertèbres, les deux faces de base de l'entretoise étant placées au contact des corps vertébraux. L'entretoise comporte en outre une cavité ouverte dans laquelle il est possible de placer un greffon osseux ou analogue dans le but de souder entre eux les deux corps vertébraux par ostéosynthèse.

Bien que cette réalisation donne généralement de bons résultats, elle peut, dans des circonstances exceptionnelles, ne pas permettre à la cage intervertébrale de remplir le rôle pour lequel elle a été prévue.

En effet, dans les premiers temps suivant l'implantation de la cage, on constate que, lors de certains mouvements de relativement grande amplitude de la colonne vertébrale, par exemple quand la tête se penche très en arrière, il peut se créer un espace non négligeable entre les faces de base de l'entretoise et les vertèbres, espace par lequel le greffon ou une partie de celui-ci peut s'échapper de la cavité dans laquelle il était placé. Si c'est le cas, le greffon ne peut plus alors jouer correctement son rôle et une nouvelle intervention chirurgicale est nécessaire.

Pour tenter de pallier les inconvénients mentionnés ci-dessus, il a été conçu des cages comportant une lame d'ancrage pivotant sur une face de l'entretoise. Une telle cage est par exemple décrite dans le document US-A-5 683 394. Cette réalisation donne théoriquement de bons résultats mais présente parfois des insuffisances, notamment quant au maintien de la patte d'ancrage dans sa position de blocage.

C'est ainsi qu'il a été aussi conçu une cage intervertébrale comme celle qui est décrite dans le document EP-A-1 104 665, qui donne de bons résultats. Cependant, la présente invention a pour but de réaliser un perfectionnement à ce type de cage décrit dans ces deux documents antérieurs.

Plus précisément, la présente invention a pour objet une cage intervertébrale pour le traitement du rachis dégénératif, comprenant:
une entretoise sensiblement en forme de disque comportant un logement creux, ce disque comportant deux faces de base opposées sensiblement planes et parallèles et une paroi latérale reliant les deux faces de base, cette paroi latérale comportant deux parties opposées, ladite entretoise étant apte à être disposée entre les faces en regard des deux corps vertébraux respectivement de deux vertèbres consécutives, en remplacement du disque endommagé entre ces deux vertèbres, les deux faces de base de l'entretoise étant aptes à être placées au contact au moins partiel des deux corps vertébraux,
   - au moins une lame, la lame comportant deux extrémités opposées conformées en biseau, et
   - des moyens pour déplacer ladite lame par rapport à une première des deux parties de paroi latérale opposées de façon que la lame soit apte à prendre deux positions,
      * une première position dans laquelle la lame est entièrement située dans l'espace compris entre les deux premier et second plans contenant les deux faces de base de l'entretoise, et
      * une seconde position dans laquelle les deux extrémités opposées de ladite lame émergent de part et d'autre dudit espace,
caractérisée par le fait que ladite lame comporte une surface d'épaulement bordant au moins partiellement chacune de ses deux extrémités, les deux surfaces d'épaulement étant agencées pour venir au contact respectivement des faces en regard des deux corps vertébraux quand ladite lame est dans sa seconde position et quand ses deux extrémités sont enfoncées respectivement dans lesdits deux corps vertébraux.

D'autres caractéristiques et avantages de l'invention apparaîtront au cours de la description suivante donnée en regard des dessins annexés à titre illustratif mais nullement limitatif, dans lesquels :
La figure 1 représente une vue en coupe longitudinale d'un premier mode de réalisation d'une cage intervertébrale selon l'invention, comportant une entretoise et une lame d'une première structure donnée,
La figure 2 représente une vue en coupe d'une partie de la cage représentant essentiellement la lame selon la figure 1, cette coupe étant référencée II-II sur la figure 1,
Les figures 3 et 4 représentent deux vues, respectivement de côté et de face, d'un deuxième mode de réalisation de la lame entrant dans la composition de la cage selon l'invention, et
La figure 5 représente une demi-vue en coupe partielle d'un autre mode de réalisation de la cage selon l'invention illustrant essentiellement un troisième mode de réalisation de la lame, le mode de réalisation de l'entretoise étant sensiblement identique à celui illustré sur la figure 1.

Il est bien précisé que, sur les figures, les mêmes références désignent les mêmes éléments, quelle que soit la figure sur laquelle elles apparaissent et quelle que soit la forme de représentation de ces éléments. De même, si des éléments ne sont pas spécifiquement référencés sur l'une des figures, leurs références peuvent être aisément retrouvées en se reportant à une autre figure.

Le Demandeur tient aussi à préciser que les figures représentent plusieurs modes de réalisation de l'objet selon l'invention, mais qu'il peut exister d'autres modes de réalisation qui répondent à la définition de cette invention.

Il précise en outre que, lorsque, selon la définition de l'invention, l'objet de l'invention comporte "au moins un" élément ayant une fonction donnée, le mode de réalisation décrit peut comporter plusieurs de ces éléments.

Il précise aussi que, si les modes de réalisation de l'objet selon l'invention tel qu'illustré comportent plusieurs éléments de fonction identique et que si, dans la description, il n'est pas spécifié que l'objet selon cette invention doit obligatoirement comporter un nombre particulier de ces éléments, l'objet de l'invention pourra être défini comme comportant "au moins un" de ces éléments.

La présente invention concerne une cage intervertébrale pour, notamment, le traitement du rachis dégénératif.

Cette cage intervertébrale comprend essentiellement une entretoise 1 en forme de disque sensiblement parallélépipédique comportant un logement en creux 2.

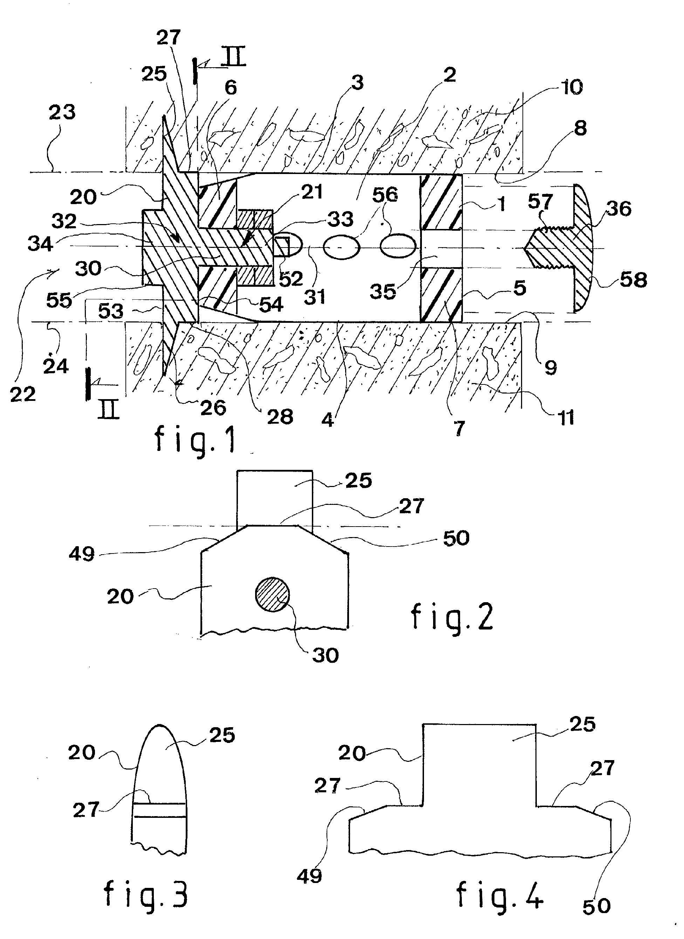

De ce fait, ce disque comporte deux faces de base opposées 3, 4 sensiblement planes et parallèles entre elles et une paroi latérale 5 reliant les deux faces de base. Dans une forme particulièrement avantageuse, cette paroi latérale comporte deux parties opposées 6, 7, une première partie 6 étant d'une hauteur légèrement inférieure à la seconde partie 7, comme représenté sur la figure 1.

L'entretoise 1 est apte à être disposée entre les faces en regard 8, 9 des deux corps vertébraux 10, 11 respectivement de deux vertèbres consécutives, en remplacement du disque endommagé qui se trouvait entre ces deux vertèbres, les deux faces de base 3, 4 de l'entretoise 1 étant aptes à être placées au contact au moins partiel des faces en regard des deux corps vertébraux.

La cage comporte en outre au moins une lame 20 et des moyens 21 pour déplacer cette lame par rapport à la première des deux parties opposées 6, 7 de la paroi latérale 5, en l'occurrence la partie 6 sur la figure 1, de façon que la lame puisse prendre deux positions:
- une première position dans laquelle la lame est entièrement située dans l'espace 22 compris entre les deux premier et second plans 23, 24 contenant les deux faces de base 3, 4 de l'entretoise 1, et
- une seconde position comme celle qui est illustrée sur la figure 1, dans laquelle les deux extrémités opposées 25, 26 de la lame émergent de part et d'autres de cet espace 22.

En outre, de façon avantageuse, les deux extrémités 25, 26 de la lame 20 sont conformées en biseau de façon à pouvoir pénétrer plus facilement dans l'os des corps vertébraux 10, 11 à la manière d'une lame de couteau.

Selon une caractéristique de l'invention, la lame comporte une surface d'épaulement 27, 28 bordant au moins partiellement chacune de ses deux extrémités 25, 26, ces surfaces d'épaulement étant en outre agencées sur la lame pour venir au contact respectivement des faces en regard 8, 9 des deux corps vertébraux 10, 11 quand la lame est dans sa seconde position et que ses deux extrémités 26, 27 sont enfoncées dans la partie osseuse respectivement des deux corps vertébraux 10, 11.

Sur les figures 1 et 2, sont illustrées deux surfaces d'épaulement 27, 28 situées en déport du plan contenant la lame 20. Cependant, il est bien évident qu'elles peuvent entourer, en totalité ou partiellement, ces deux extrémités.

A titre d'exemple illustratif, les figures 3 et 4 représentent un autre mode de réalisation de la lame 20. Dans cette réalisation, la lame comporte deux portions de surface d'épaulement 27 pour une extrémité 25 de la lame, ces deux portions étant situées de part et d'autre de l'extrémité de la lame, sensiblement dans le plan de la lame.

La forme et la structure de ces surfaces épaulement seront définies en fonction de l'état pathologique des vertèbres à soigner et en fonction des besoins du praticien réalisant l'intervention pour qu'il puisse rétablir les vertèbres dans leur position originelle, lorsque le disque intervertébral qui les séparait était sain et assurait sa fonction physiologique.

De plus, comme représenté sur la figure 1, la distance séparant les deux surfaces épaulement 27, 28 est légèrement supérieure à la hauteur de la première partie 6 de la paroi latérale 5 prise selon une perpendiculaire aux premier et second plans 23, 24 définis auparavant.

De cette façon, les deux corps vertébraux 10, 11 dans lesquels sont ancrées les deux extrémités opposées 25, 26 de la lame 20, reposent sur les deux surfaces d'épaulement 27, 28 au lieu de reposer sur la partie des faces de base bordant la première partie 6 de la paroi latérale 5.

En effet, dans une réalisation particulièrement avantageuse, la lame 20 est réalisée en un matériau métallique, par exemple du titane ou de l'acier biocompatible, et constitue de ce fait un pilier d'appui plus important que la partie des faces de base bordant la première partie 6 de la paroi latérale 5, sachant qu'en général l'entretoise est réalisée dans un matériau plastique. Même si ce matériau plastique peut être relativement rigide et solide, il ne le sera jamais autant que le métal dans lequel aura été réalisée la lame. De cette façon, la lame constitue un pilier de renfort à une extrémité de l'entretoise permettant de bien assurer le maintien à distance des deux corps vertébraux 10, 11 et de rétablir la distance intervertébrale originelle lorsque le disque intervertébral présent entre les deux corps vertébraux était sain.

Dans une réalisation avantageuse, les moyens 21 définis ci-dessus, pour déplacer la lame 20 par rapport à une première des deux parties de paroi latérale opposées, en l'occurrence la partie 6 dans la représentation sur la figure 1, de façon que la lame soit apte à apprendre deux positions, comportent un arbre 30 monté au moins en rotation, et avantageusement en outre en translation, suivant un premier axe 31, dans la première partie 6 de la paroi latérale 5, et des moyens 32 pour monter la lame en coopération avec une première extrémité 34 de l'arbre 30, la seconde extrémité 33 de l'arbre plongeant dans le logement creux 2.

Cet arbre 30 permet de transmettre la commande pour faire passer la lame 20 de sa première position à sa seconde position, et réciproquement s'il est par exemple nécessaire de retirer la cage intervertébrale après qu'elle ait été implantée.

Dans une réalisation avantageuse, le logement en creux 2 défini ci-dessus est réalisé sensiblement au centre de l'entretoise et débouche sur les deux faces de base opposées 3, 4 de l'entretoise de façon que le greffon osseux qu'il peut contenir favorise l'ostéosynthèse entre les corps vertébraux 10, 11 des deux vertèbres consécutives.

Avantageusement, la paroi latérale 5 reliant les deux faces de base opposées 3, 4 comportent, elle aussi, des percées 56 permettant d'obtenir une ostéosynthèse latérale afin d'enrober complètement la cage intervertébrale qui a été implantée.

Dans le cas de la réalisation d'un logement en creux 2 comme défini ci-dessus, il est avantageux que la seconde partie 7 de paroi latérale 5 comporte un orifice 35 centré sur le premier axe 31. Il est ainsi possible d'implanter la cage intervertébrale de façon que lame 20 soit située, ou bien dans la partie antérieure des vertèbres, ou bien dans la partie postérieure.

Quand la cage intervertébrale est implantée entre deux vertèbres et que la lame se trouve dans la partie postérieure de la colonne vertébrale, il est possible de la faire passer de sa première position à sa seconde position au moyen d'un ancillaire qui vient coopérer directement avec elle, par exemple par une partie en saillie 55 réalisée sur la face 53 de la lame 20 opposée à celle 54 qui est en regard de la première partie 6 de paroi latérale 5.

En revanche, lorsque que la cage intervertébrale est implantée de façon que la lame 20 soit positionnée dans la partie antérieure de la colonne vertébrale, il est bien évident que la voie antérieure pour atteindre la lame et la faire pivoter comme décrit ci-avant est compliquée. Il est donc préférable, dans ce cas également, de pouvoir intervenir par voie postérieure. La présence de l'orifice 35 permet alors d'utiliser un ancillaire qui peut coopérer avec l'extrémité 33 de l'arbre 30 qui plonge dans le logement creux 2, en passant par cet orifice 35.

Les moyens pour faire passer, par rotation, la lame 20 de sa première position à sa seconde position et réciproquement, peuvent être constitués par des parties en saillie de forme polygonale, comme celle qui est schématisée en 55 sur la face 53 de la lame et celle qui est illustrée 52 à l'extrémité 33 de l'arbre 30. Avec ces parties en saillie 52, 55, peuvent coopérer des ancillaires sensiblement identiques à des outils classiques que l'on dénomme couramment : clés à tubes, tourne vis ou analogues.

Selon une autre caractéristique de l'invention, la cage intervertébrale comporte un bouchon 36. Un tel bouchon comporte de façon classique un embout 57 solidaire d'une tête d'épaulement 58, l'embout pouvant s'enficher en force dans l'orifice 35 tandis que la tête d'épaulement vient se plaquer contre la surface externe de la seconde partie 7 de la paroi latérale 5. Avantageusement, l'embout 57 comporte, comme illustré schématiquement, des dents d'insert ou analogues.

Dans une réalisation avantageuse, ce bouchon 36 est réalisé en un matériau radio-opaque et avantageusement rigide, par exemple du métal tel que du titane, de l'acier biocompatible, etc..

Ce bouchon présente deux avantages pour la cage selon l'invention. L'un de ces avantages est de constituer un second repère radio-opaque lors d'une radiographie et, en combinaison avec la lame 2, de déterminer la position de la cage quand elle est implantée. L'autre avantage est de constituer un renfort important de la seconde partie 7 de la paroi latérale 5 et donc un pilier de renfort et de support qui, avec celui que constitue la lame comme décrite ci-avant, confère à la cage intervertébrale une rigidité qui contribue à la réalisation d'une ostéosynthèse de très bonne qualité entre les deux corps vertébraux consécutifs 10, 11, puisque la distance entre ces deux corps vertébraux ne peut pas varier notablement, au contraire de celle qui est définie par les cages intervertébrales selon l'art antérieur.

La lame 20 peut prendre plusieurs formes. Les figures 1 à 4 illustrent deux modes de réalisation de cette lame, dans lesquels la lame est d'une seule pièce.

Dans ces deux modes de réalisation, les moyens 32 définis ci-avant pour monter la lame en coopération avec la première extrémité 34 de l'arbre 30 sont essentiellement constitués par des moyens de solidarisation de cette première extrémité 34 de l'arbre avec la lame 20 et de façon que la lame soit située dans un plan sensiblement perpendiculaire au premier axe 31.

Dans une réalisation avantageuse, la lame 20 et l'arbre 30 sont réalisés en une seule pièce par exemple en titane par usinage. De cette façon, en agissant sur l'extrémité 33 de l'arbre 30 situé dans le logement en creux 2 ou sur la partie en saillie 55 de la lame 20, il est possible de faire subir à la lame une rotation autour de l'axe 31. Dans cette rotation, lorsque la lame passe de sa première position à sa seconde position, ses deux extrémités en biseau 25, 26 pénètrent dans les corps vertébraux 10, 11 et s'y ancre jusqu'à ce que les deux surfaces d'épaulement 27, 28 viennent se plaquer contre les faces en regard 8, 9 des deux corps vertébraux.

Lorsque la lame est réalisée en une seule pièce comme décrit ci-dessus, il est avantageux que chaque surface d'épaulement 27, 28 soit réalisée en continuité d'une rampe 49, 50, figures 2 à 4. Cette rampe glisse sur la face du corps vertébral lors de l'introduction de la lame 20 dans ce corps vertébral lorsqu'elle passe de sa première position à sa seconde position par une rotation autour du premier axe 31, tout en exerçant une légère distraction entre les deux corps vertébraux pour les écarter et les ramener à leur position originelle, et ainsi favoriser le positionnement des deux surfaces d'épaulement pour qu'elles s'appliquent à plat sur les deux faces en regard des deux corps vertébraux.

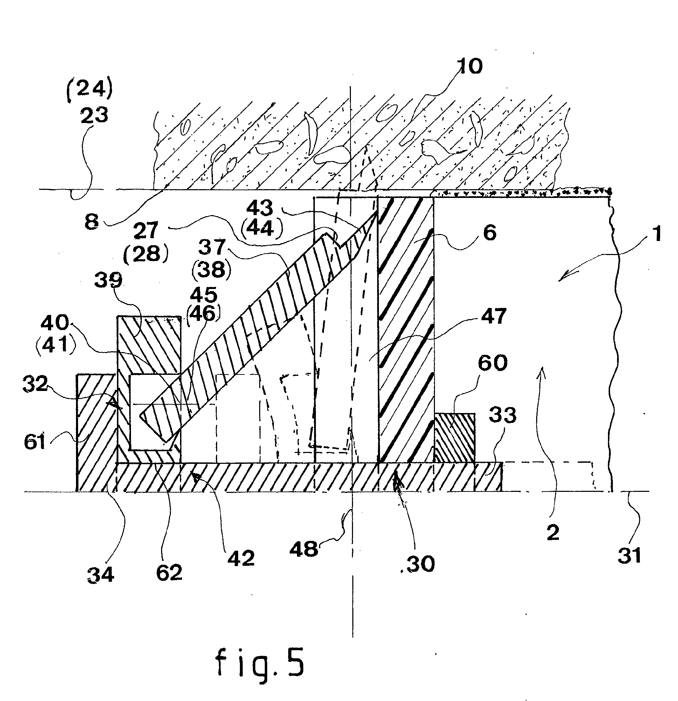

La figure 5 représente partiellement une demi-partie, selon la règle conventionnelle du dessin industriel, d'un troisième mode de réalisation de la cage intervertébrale et plus particulièrement de la lame 20. Dans cette réalisation, la lame 20 est constituée de deux pattes 37, 38 et d'une tête de maintien 39 de ces deux pattes. Les deux pattes sont montées, par une de leurs extrémités 45, 46, en rotation sur la tête de maintien 39 sensiblement autour respectivement d'un deuxième et d'un troisième axes 40, 41 sensiblement parallèles aux premier et second plans 23, 24 et perpendiculaires au premier axe 31 défini ci-dessus.

Dans le mode de réalisation illustré sur cette figure 5, les deux pattes sont serties sur la tête de maintien 39, la rotation étant obtenue par la déformation plastique du matériau constituant cette tête de maintien. Il peut cependant exister d'autres modes de réalisation, par exemple un ensemble "arbre-palier".

Les moyens 32 pour monter la lame en coopération avec la première extrémité 34 de l'arbre 30 sont constitués par des moyens 42 pour exercer une traction sur la tête de maintien 39 par rapport à l'entretoise 1, et plus particulièrement dans le mode de réalisation illustré, par rapport à la première partie 6 de paroi latérale 5.

Ces moyens 42 sont essentiellement constitués par une tête d'épaulement 61 solidaire de l'extrémité 34 de l'arbre 30, cet arbre traversant la pièce de maintien 39 par une percée centrale 62 de façon que la pièce de maintien soit au contact de la tête d'épaulement 61 et située entre cette tête d'épaulement et la première partie 6 de la paroi latérale 5 de l'entretoise 1.

En effectuant, par exemple, un vissage d'un écrou 60 sur l'extrémité 33 de l'arbre 30 plongeant dans le logement 2, il est alors possible de tirer sur la tête d'épaulement 61 et d'entraîner la tête de maintien 39 pour la rapprocher de la première partie 6 de paroi latérale 5.

Dans un autre mode de réalisation, l'écrou 60 peut être fixe par rapport à l'entretoise 1, la translation de l'arbre 30 étant obtenue par son vissage dans cet écrou en agissant par exemple sur la tête d'épaulement 61.

Dans le mouvement de translation de l'arbre 30, les deux pattes 37, 38 qui étaient initialement positionnées en oblique comme représenté en traits continus sur la figure 5, en formant entre elles par exemple un angle d'environ quatre-vingt-dix degrés, s'écartent l'une de l'autre par augmentation de cet angle pour se positionner, en finale, suivant un angle sensiblement plat comme illustré en traits interrompus sur cette figure 5. Dans ce mouvement, les deux extrémités en biseau 43, 44 des deux pattes 37, 38 viennent s'ancrer par pénétration dans les parties osseuses des deux corps vertébraux 10, 11, jusqu'à ce que les deux surfaces d'épaulement 27, 28 butent contre les faces en regard 8, 9 de ces corps vertébraux.

Dans cette réalisation, les extrémités des deux pattes, c'est-à-dire de la lame 20, sont soumises à une translation, alors que, dans les réalisations illustrées sur les figures 1 à 4 elles sont soumises à des mouvements de rotation.

Dans le mode de réalisation selon la figure 5, il est avantageux que les deux extrémités 43, 44 des deux pattes 37, 38 opposées à celles 45, 46 qui sont montées en rotation sur la tête de maintien 39, soient montées coulissantes en translation dans une rainure 47 réalisée dans la première partie 6 de la paroi latérale 5. Cette rainure 47 est réalisée dans l'entretoise suivant un quatrième axe 48 sensiblement perpendiculaire aux premier et second plans 23, 24.

Les deux extrémités 43, 44 des deux pattes 37, 38 étant montées coulissantes dans cette rainure 47, elles s'écartent l'une de l'autre en restant constamment dans un même plan qui est défini par les premier et quatrième axes 31, 48. Elles ne peuvent donc pas pivoter autour de l'axe 31 lors du vissage, ou de l'écrou 60 ou de l'arbre 30.

Il est aussi avantageux que les deux surfaces d'épaulement 27, 28 soient sensiblement planes et parallèles entre elles, et, lorsque les extrémités 43, 44 des deux pattes sont ancrées dans les corps vertébraux 10, 11, sensiblement parallèles au plan des faces en regard 8, 9 de ces corps vertébraux.

En plus de pouvoir maintenir la distance entre les deux corps vertébraux, ces surfaces d'épaulement réalisées par des surfaces planes et parallèles entre elles permettent de maintenir la lame dans sa seconde position et de l'empêcher de revenir à sa première position sous l'effet de quelque contrainte que ce soit qui serait exercée par le patient lui-même, tout en lui permettant de revenir à cette première position sous l'effet de forces extérieures exercées, par exemple, à l'aide d'un instrument ancillaire.

## Revendications

1. Cage intervertébrale pour le traitement du rachis dégénératif, comprenant:
une entretoise (1) sensiblement en forme de disque comportant un logement creux (2), ce disque comportant deux faces de base opposées (3, 4) sensiblement planes et parallèles et une paroi latérale (5) reliant les deux faces de base, cette paroi latérale comportant deux parties opposées (6, 7), ladite entretoise étant apte à être disposée entre les faces en regard (8, 9) des deux corps vertébraux (10, 11) respectivement de deux vertèbres consécutives, en remplacement du disque endommagé entre ces deux vertèbres, les deux faces de base de l'entretoise étant aptes à être placées au contact au moins partiel des deux corps vertébraux,
- au moins une lame (20), ladite lame comportant deux extrémités opposées (25, 26) conformées en biseau, et
- des moyens (21) pour déplacer ladite lame par rapport à une première (6) des deux parties de paroi latérale de façon que la lame soit apte à prendre deux positions :
* une première position dans laquelle la lame est entièrement située dans l'espace (22) compris entre les deux premier et second plans (23, 24) contenant les deux faces de base (3, 4) de l'entretoise (1), et
* une seconde position dans laquelle les deux extrémités opposées (25, 26) de ladite lame émergent de part et d'autre dudit espace (22),
**caractérisée par le fait que** ladite lame comporte une surface d'épaulement (27, 28) bordant au moins partiellement chacune de ses deux extrémités (25, 26), les deux surfaces d'épaulement étant agencées pour venir au contact respectivement des faces en regard (8, 9) des deux corps vertébraux (10, 11) quand ladite lame (20) est dans sa seconde position et quand ses deux extrémités (26, 27) sont enfoncées respectivement dans lesdits deux corps vertébraux (10, 11).

2. Cage intervertébrale selon la revendication 1, **caractérisée par le fait que** la distance séparant les deux surfaces d'épaulement (27, 28) est légèrement supérieure à la hauteur de la première (6) des deux parties de paroi latérale, prise selon une perpendiculaire aux deux premier et second plans (23, 24).

3. Cage intervertébrale selon l'une des revendications 1 et 2, **caractérisée par le fait que** les moyens (21) pour déplacer ladite lame par rapport à une première des deux parties de paroi latérale de façon que la lame soit apte à prendre deux positions, comportent un arbre (30) monté au moins en rotation suivant un premier axe (31) dans ladite première partie (6) de paroi latérale, des moyens (32) pour monter ladite lame en coopération avec une première extrémité (34) de l'arbre (30), la seconde extrémité (33) dudit arbre plongeant dans le logement en creux (2).

4. Cage intervertébrale selon la revendication 3, **caractérisée par le fait qu'**elle comporte un orifice (35) réalisé dans la seconde partie (7) de paroi latérale (5), ledit orifice étant centré sur ledit premier axe (31).

5. Cage intervertébrale selon la revendication 4, **caractérisée par le fait qu'**elle comporte un bouchon (36) pour boucher ledit orifice.

6. Cage intervertébrale selon l'une des revendications 3 à 5, **caractérisée par le fait que**, ladite lame (20) étant réalisée en une seule pièce, les moyens (32) pour monter ladite lame en coopération avec une première extrémité (34) dudit arbre (30) sont des moyens de solidarisation de la première extrémité (34) de l'arbre avec ladite lame (20) de façon que ladite lame soit située dans un plan sensiblement perpendiculaire audit premier axe (31).

7. Cage intervertébrale selon l'une des revendications 3 à 5, **caractérisée par le fait que**, ladite lame (20) étant constituée de deux pattes (37, 38) et d'une tête de maintien (39) de ces deux pattes, les deux pattes étant montées, par une de leurs extrémités (45, 46), en rotation sur ladite tête de maintien (39) sensiblement autour respectivement d'un deuxième et d'un troisième axes (40, 41) sensiblement parallèles aux premier et second plans (23, 24) et perpendiculaires au premier axe (31), les moyens (32) pour monter ladite lame en coopération avec une première extrémité (34) dudit arbre (30) sont constitués par des moyens (42) pour exercer une traction sur la tête de maintien (39) par rapport à l'entretoise (1).

8. Cage intervertébrale selon la revendication 7, **caractérisée par le fait que** les deux extrémités (43, 44) des deux pattes (37, 38) opposées à celles (45, 46) qui sont montées en rotation sur ladite tête de maintien (39) constituent les deux extrémités (25, 26) de ladite lame (20) et sont montées coulissantes en translation dans une rainure (47) réalisée dans ladite première partie (6) de paroi latérale (5) suivant un quatrième axe (48) sensiblement perpendiculaire aux deux premier et second plans (23, 24).

9. Cage intervertébrale selon l'une des revendications 5 à 8, **caractérisée par le fait que** l'entretoise (1) est en un matériau plastique et que la lame (20) et le bouchon (36) sont en un matériau radio-opaque.

10. Cage intervertébrale selon la revendication 9, **caractérisée par le fait que** le matériau radio-opaque est du métal.

11. Cage intervertébrale selon l'une des revendications 1 à 10, **caractérisée par le fait que** les deux surfaces d'épaulement (27, 28) sont sensiblement planes et parallèles.

12. Cage intervertébrale selon la revendication 11, **caractérisée par le fait qu'**elle comporte au moins une rampe (49, 50) réalisée en continuité de chaque surface d'épaulement
